# EUROPÄISCHE PATENTSCHRIFT

(11) **EP 2 942 623 B1**
(45) Veröffentlichungstag und Bekanntmachung des Hinweises auf die Patenterteilung: **20.01.2021**
(21) Anmeldenummer: 15166441.4
(22) Anmeldetag: 05.05.2015
(51) Int. Cl.: G01N 33/48, G01N 33/58, A61B 5/1455, G01N 21/00, G01N 33/543

(54) **DETEKTIONSVORRICHTUNG ZUR ANREICHERUNG VON PROBENMATERIAL**
DETECTION DEVICE FOR ENRICHMENT OF SAMPLE MATERIAL
DISPOSITIF DE DÉTECTION DESTINÉ À AMÉLIORER UN MATÉRIAU D'ÉCHANTILLON

(30) Priorität: 09.05.2014 DE 102014006906
(43) Veröffentlichungstag der Anmeldung: 11.11.2015
(73) Patentinhaber: GILUPI GmbH, 14473 Potsdam (DE)
(72) Erfinder: Krusekopf, Solveigh, 13158 Berlin (DE); Niestroj-Pahl, Robert, 10249 Berlin (DE); Lücke, Klaus, 14476 Potsdam (DE); Steinbrück, Dörte, 14469 Potsdam (DE); Sass, Stefan, 14467 Potsdam (DE); Löhmannsröben, Hans-Gerd, 38440 Wolfsburg (DE); Geßner, André, 14476 Potsdam-Golm (DE)
(74) Vertreter: Grünecker Patent- und Rechtsanwälte PartG mbB

(56) Entgegenhaltungen:
- WO-A1-2010/145824
- US-A1- 2011 082 354
- MASTICHIADIS C ET AL: "Capillary-based immunoassays, immunosensors and DNA sensors - steps towards integration and multi-analysis", TRAC, TRENDS IN ANALYTICAL CHEMISTRY, ELSEVIER, AMSTERDAM, NL, Bd. 27, Nr. 9, 1. Oktober 2008 (2008-10-01), Seiten 771-784, XP025559772, ISSN: 0165-9936, DOI: 10.1016/J.TRAC.2008.08.003 [gefunden am 2008-09-17]
- Li, Amstutz III, Tang, Hang, Zhu, Zhang Shelton, Karns: "Integrating waveguide biosensor" In: Rasooly and Herold: "Biosensors and Biodetection", 2010, Humana Press, XP9184879, Bd. 503, Seiten 389-401, * Zusammenfassung; S. 390-391; Fig. 1, 3; S. 398, letzer Absatz -S. 400; ganzes Dokument *
- LIGLER F S ET AL: "INTEGRATING WAVEGUIDE BIOSENSOR", ANALYTICAL CHEMISTRY, AMERICAN CHEMICAL SOCIETY, US, Bd. 74, Nr. 3, 1. Februar 2002 (2002-02-01), Seiten 713-719, XP001115843, ISSN: 0003-2700, DOI: 10.1021/AC015607S
- FRANCES S. LIGLER: "Perspective on Optical Biosensors and Integrated Sensor Systems", ANALYTICAL CHEMISTRY, Bd. 81, Nr. 2, 15. Januar 2009 (2009-01-15), Seiten 519-526, XP055196023, ISSN: 0003-2700, DOI: 10.1021/ac8016289
- Izabela Firkowska ET AL: "Biocompatible Nanomaterials and Nanodevices Promising for Biomedical Applications" In: "Nanomaterials for Application in Medicine and Biology", 1 January 2008 (2008-01-01), Springer Netherlands, Dordrecht, XP055119345, ISSN: 1874-6500 ISBN: 978-1-40-206829-4 pages 1-15, DOI: 10.1007/978-1-4020-6829-4_1, * the whole document *

## Beschreibung

Die Erfindung betrifft eine Detektionsvorrichtung zur in vitro Anreicherung von Probenmaterial, umfassend eine mit Detektionsrezeptoren bestückte Funktionsfläche.

Viele Zelltypen, Moleküle, Tumor- und Biomarker sind in Körperflüssigkeiten zwar vorhanden, können aber oft wegen ihrer niedrigen Konzentration nicht effizient genug durch die herkömmlichen Methoden der Anreicherung gewonnen werden, um anschließend in etablierten diagnostischen Verfahren der klinischen Chemie, Pathologie oder Zytologie genutzt werden zu können.

Zum Beispiel ist das Anreichern von speziellen Zellen, insbesondere zirkulierenden Tumorzellen, aus einer Blutprobe (in vitro) mittels kommerziell erhältlicher paramagnetischer Nanopartikel und/oder Dichtegradientenzentrifugation möglich, jedoch in nur sehr begrenzter Anzahl und mit dem Nachteil, dass die Nanopartikel an oder in der Zelle binden und diese damit schädigen bzw. die Diagnostik erschweren können. Eine dieser kommerziellen Methoden spiegelt sich in einem Test wieder, in dem z.B. zirkulierende Tumorzellen aus 7,5 ml Blutvolumen mittels paramagnetischer Nanopartikel angereichert werden, um dann Aussagen über den Krankheitsverlauf machen zu können.

Der limitierende Faktor dieser Methode ist das gewonnene Probevolumen, welches beim Anwenden einer Detektionsvorrichtung zur Anreicherung von Probenmaterial, z. B. eines funktionalisierten Katheter, um ein Vielfaches höher ist. Gefäßkatheter für die Anwendung medizinischer Interventionen sind meist zylinderförmig konstruiert. Eine derartige Detektionsvorrichtung ist beispielsweise aus der WO 2010/145824 A1 bekannt.

MASTICHIADIS C ET AL: "Capillary-based immunoassays, immunosensors and DNA sensors - steps towards integration and multi-analysis",TRAC, TRENDS IN ANALYTICAL CHEMISTRY, ELSEVIER, AMSTERDAM, NL, Bd. 27, Nr. 9, 1. Oktober 2008 (2008-10-01), Seiten 771-784, offenbart Dextranmodifizierte Oberflächen als Beschichtung für Antikörper. US 2011/082354 A1 offenbart Agarose Gel zur Verwendung als Funktionsschicht.

Der Erfindung liegt die Aufgabe zugrunde, eine Detektionsvorrichtung anzugeben, mittels der die Menge und/oder die Spezifität gewonnenen Probenmaterials mit verringertem Aufwand ermittelt werden kann.

Um die der Erfindung zugrunde liegende Aufgabe zu lösen, wird eine Detektionsvorrichtung zur in vitro Anreicherung von Probenmaterial gemäß Anspruch 1 bereit gestellt, sowie eine Verwendung nach Anspruch 14 und ein Verfahren nach Anspruch 15.

Dadurch ermöglicht die erfindungsgemäße Detektionsvorrichtung, das gewonnene Probenmaterial zeitsparend und mit geringem Aufwand optisch zu messen, insbesondere mittels Fluoreszensmessung, und hierdurch die Anzahl und/oder die Spezifität der durch die Detektionsrezeptoren gewonnenen Zielmoleküle beziehungsweise Zielzellen zu ermitteln. Eine zeitaufwändige Auszählung und/oder Bestimmung gewonnener Zielzellen kann somit entfallen oder verkürzt werden. Mit nur geringem Aufwand kann somit bestimmt werden, ob die jeweiligen Zielzellen in ausreichender Anzahl gebunden worden sind und die Probeentnahme damit erfolgreich war, oder ob weitere Zielzellen für eine ausreichend sichere Diagnostik erforderlich sind.

Ebenso gewährleistet die erfindungsgemäße Detektionsvorrichtung, dass mittels optischer Messung, insbesondere Fluoreszensmessung, spezifische Zielzellen beziehungsweise Zielmoleküle von unspezifischen Zellen unterschieden werden. Dies kann beispielsweise dadurch bewerkstelligt werden, dass nach der Probeentnahme spezifische Zielzellen und unspezifische Zellen unterschiedlich markiert werden, sodass durch Fluoreszensmessung eine Unterscheidung möglich ist. Nach Probeentnahme und optischer Messung kann das Probenmaterial dann den jeweils erforderlichen Diagnoseverfahren zugeführt werden.

Die Funktionsfläche kann mit chemisch identischen oder chemisch verschiedenen Detektionsrezeptoren bestückt sein. Somit können bei einer Anwendung nach Bedarf auch verschiedene Liganden beziehungsweise Zielzellen angereichert werden. Im Sinne dieser Erfindung werden alle Strukturen, insbesondere Rezeptoren oder Liganden, die geeignet sind, Zielmoleküle und Zielzellen einzufangen, als Detektionsrezeptoren bezeichnet. Ferner werden alle Zielmoleküle und Zielzellen, die an den Detektionsrezeptoren andocken können, vereinfachend als Liganden bezeichnet. Der Begriff Probenflüssigkeit bezeichnet eine in flüssiger Form vorliegende Probe.

Weiterhin wird im folgenden unter "proximal" die dem jeweiligen Bediener der Detektionsvorrichtung zugewandte Seite verstanden, wobei unter "distal" die dem jeweiligen Patienten zugewandete Seite verstanden werden soll.

Bevorzugte Weiterbildungen der Erfindung sind Gegenstände der Unteransprüche.

Es kann sich als hilfreich erweisen, wenn die erfindungsgemäße Detektionsvorrichtung, insbesondere deren Funktionsfläche, gemäß der in der WO 2010/145824 A1 beschriebenen Vorrichtung ausgebildet ist, mit der Maßgabe, dass eine erfindungsgemäße Messstruktur vorgesehen ist. Der Offenbarungsgehalt der WO 2010/145824 A1 soll Bestandteil der vorliegenden Patentanmeldung sein.

Weiterhin kann es von Vorteil sein, wenn die Messstruktur einen Lichtwellenleiter aus einem Kunststoffmaterial aufweist und/oder dass die Messstruktur eine optische Faser, vorzugsweise eine polymere optische Faser aufweist. Insbesondere kann die optische Faser aus einem nicht-fluoreszierendem Material besteht. Beispielsweise kann die optische Faser aus CYTOP, Fontex® oder PMMA bestehen.

Gemäß einer vorteilhaften Ausgestaltung der Detektionsvorrichtung kann die Messstruktur eine einzelne oder mehrere miteinander verbundene und/oder verdrehte Fasern aufweisen. Ebenso kann die Messstruktur einen durchgängigen Kern bilden. Zudem ist es möglich, dass die Messstruktur als Träger für die Funktionsfläche ausgebildet ist. Schließlich kann auch die Messstruktur zusätzlich zu einem gesonderten Träger für die Funktionsfläche ausgebildet sein, beispielsweise zusätzlich zu einem metallischen Führungsdraht. Vorzugsweise wird jedoch ein herkömmlicher Führungsdraht vollständig durch eine als Führungselement ausgebildete Messstruktur ersetzt, was eine besonders präzise Messung von Zielmolekülen bei gleichzeitig geringer Anzahl an Vorrichtungskomponenten ermöglicht.

Weiterhin kann es hilfreich sein, wenn die Messstruktur an ihrem proximalen Ende einen Anschluss für ein optisches Messgerät, vorzugsweise für ein fasergekoppeltes Spektrometer oder eine Photodiodeneinheit aufweist. Ebenso kann es von Vorteil sein, wenn die Messstruktur an ihrem proximalen Ende abschnittsweise von einer Halterung umgeben ist.

Gemäß einer noch weiteren vorteilhaften Ausgestaltung der Detektionsvorrichtung umfassen die Detektionsrezeptoren Antikörper, Antikörperfragmente, Aminosäuren-Strukturen, Nukleinsäure-Strukturen und/oder synthetische Strukturen mit spezifischer Affinität zu Oberflächen von Zielzellen, vorzugsweise monoklonale Antikörper murinen Ursprungs, chimäre Antikörper oder humanisierte Antikörper, bevorzugt anti-CD146 und/oder anti-EpCAM Antikörper. Weiterhin sind die Detektionsrezeptoren vorzugsweise zur Detektion von Analyten in Körperflüssigkeiten und/oder seltenen Zellen in Körperflüssigkeiten, vorzugsweise im Blut zirkulierenden endothelialen Zellen einsetzbar.

In weiterer vorteilhafter Ausgestaltung kann die Detektionsvorrichtung dadurch gekennzeichnet sein, dass sich die Funktionsfläche über einen Funktionsabschnitt erstreckt, der am distalen Ende der Detektionsvorrichtung ausgebildet ist, wobei der Funktionsabschnitt vorzugsweise eine Länge von 20-60 mm und/oder eine Dicke von 0,1 bis 1,0 mm, besonders bevorzugt von 0,5 mm aufweist. Dabei kann der Funktionsabschnitt besonders bevorzugt in eine Vene oder andere Gefäße oder Körperhöhlen einführbar sein.

Schließlich kann es hilfreich sein, wenn die mit den Detektionsrezeptoren bestückte Funktionsfläche durch eine Funktionsschicht aus blutabweisendem und/oder biokompatiblem und/oder hämokomatiblem Material gebildet ist, die entlang des Funktionsabschnitts der Detektionsvorrichtung mittelbar oder unmittelbar auf der Messstruktur angeordnet ist, wobei das hämokompatible Material vorzugsweise aus einem natürlichen Biopolymer, besonders bevorzugt aus Alginat besteht und/oder hydrophile Eigenschaften aufweist und/oder als Hydrogel ausgebildet ist.

Nach einer weiteren Ausgestaltung der Detektionsvorrichtung kann die Funktionsfläche abschnittsweise oder durchgängig mit Detektionsrezeptoren bestückt. Ebenso kann die Funktionsfläche mit Detektionsrezeptoren unterschiedlicher Art oder mit Detektionsrezeptoren unterschiedlicher Spezifitäten bestückt sein.

In noch weiter vorteilhafter Ausgestaltung können die an den Detektionsrezeptoren angereicherte Zielmoleküle mittels eines Antagonisten markierbar sein, vorzugsweise mittels eines fluoreszenzmarkierten Antikörpers. Dabei kann es weiterhin von Vorteil sein, wenn an den Detektionsrezeptoren angereicherte Zielmoleküle mittels der Messstruktur fluoreszenzspektroskopisch durch Bestimmung einer Wellenlängenverschiebung und/oder Intensitätsänderung und/oder einer Änderung der Lumineszenzabklingzeit detektierbar sind.

Die Funktionsfläche kann in vorteilhafter Ausgestaltung mit einer Schutzschicht überzogen sein, wobei die Schutzschicht vorzugsweise in Flüssigkeiten, insbesondere in Körperflüssigkeiten, vorzugsweise in Blut, löslich ist und/oder biokompatibel ist.

Die Detektionsrezeptoren können vorzugsweise über Linker oder organische funktionelle Gruppen mit der Funktionsfläche, vorzugsweise mit der Messstruktur oder der Funktionsschicht wirkverbunden sein, bevorzugt durch chemische Bindung, besonders bevorzugt durch kovalente Bindung. Insbesondere können die Detektionsrezeptoren über Carboxylgruppen an der Funktionsfläche gekoppelt sein, wobei die Kopplung der Detektionsrezeptoren vorzugsweise über Carbodiimidchemie und/oder UV-Quervernetzung erfolgt und/oder wobei die Carboxylgruppen an der Messstruktur oder innerhalb der Funktionsschicht vorgesehen sein können.

Die Detektionsrezeptoren können nachträglich an eine Funktionsfläche gekoppelt werden. Ebenso ist es möglich die Detektionsrezeptoren gemeinsam mit einer Funktionsschicht, beispielsweise aus biokompatiblem Polymer, auf der Messstruktur aufzubringen, wobei die so aufgebrachte Funktionsschicht die Funktionsfläche ausbildet.

Die Funktionsschicht kann mittels eines Haftvermittels mit der Messstruktur wirkverbunden sein. Ein derartiger Haftvermittler kann beispielsweise Aminogruppen und/oder Carboxylgruppen aufweisen. Ebenso kann die Funktionsschicht ohne Haftvermittler mit der Messstruktur wirkverbunden sein.

Ein weiterer unabhängiger Aspekt der Erfindung betrifft eine Detektionsvorrichtung nach wenigstens einer der vorangegangenen Ausführungen, hergestellt dadurch, dass zunächst eine Messstruktur mit einem Funktionsabschnitt bereitgestellt wird und anschließend eine Funktionalisierung des Funktionsabschnitts mit Detektionsrezeptoren zur Ausbildung einer Funktionsschicht, vorgenommen wird, wobei die Kopplung der Detektionsrezeptoren vorzugsweise über Carbodiimidchemie erfolgt.

In bevorzugter Weise kann eine erfindungsgemäße Detektionsvorrichtung zur invasiven Anreicherung von Probenmaterial verwendet werden, insbesondere zur Anreicherung von Zellen, umfassend bevorzugt zirkulierende Endothelzellen oder disseminierte Tumorzellen, insbesondere von hämatogen metastasierenden Tumoren, oder zur Elimination von Arzneimittel, zur Elimination von radioaktiven Tracern, zur Elimination von Magnetobeads, zur Gewinnung von Tumor- oder Biomarkern und/oder zur Elimination von Toxinen.

Noch ein weiterer unabhängiger Aspekt der Erfindung betrifft ein Verfahren zur Anreicherung von Probenmaterial unter Benutzung einer Detektionsvorrichtung nach wenigstens einer der vorangegangenen Ausführungen, wobei das Verfahren die folgenden Schritte umfasst:
a. Beaufschlagen der Funktionsfläche mit einer Probenflüssigkeit.
b. Anreicherung von Probenmaterial, vorzugsweise Zellen, DNA, RNA, Proteine, Peptide, synthetische Moleküle, an den Detektionsrezeptoren.
c. Markierung des an den Detektionsrezeptoren angereicherten Probenmaterials, vorzugsweise mit einem fluoreszenzmarkierten Antikörper.
d. Optische Detektion des Probenmaterials durch die Messstruktur mittels faseroptischer Messung, vorzugsweise Fluoreszenzmessung.

Die vorliegende Erfindung wird nachfolgend anhand eines Ausführungsbeispiels und zugehörigen Zeichnungen näher erläutert. In diesen zeigen:
- Figur 1: eine schematische Darstellung einer Detektionsvorrichtung mit einer Messtruktur vor Ausbildung einer Funktionsfläche.

- Figur 2: eine schematische Darstellung einer erfindungsgemäßen Detektionsvorrichtung mit einer Messtruktur sowie einer Funktionsfläche auf einer aufgetragenen Funktionsschicht aus Polymer.
- Figur 3: schematisch die Anbindung von Zielzellen aus Blut mittels einer erfindungsgemäßen Detektionsvorrichtung.
- Figur 4: schematisch die Markierung von gebundenen Zielzellen sowie deren optische Detektion mittels der erfindungsgemäßen Detektionsvorrichtung.

Die erfindungsgemäße Detektionsvorrichtung 1 nach dem dargestellen Ausführungsbeispiel ist ein biofunktionalisierter, medizinischer Detektionskatheter für die invasive Anreicherung von seltenen Zellen, Biomolekülen oder Arzneimitteln. Ein derartiger Detektionskatheter wird auch als medizinscher Nano-Katheter (MN-K) bzw. medical nano-catheter (MN-C) bezeichnet.

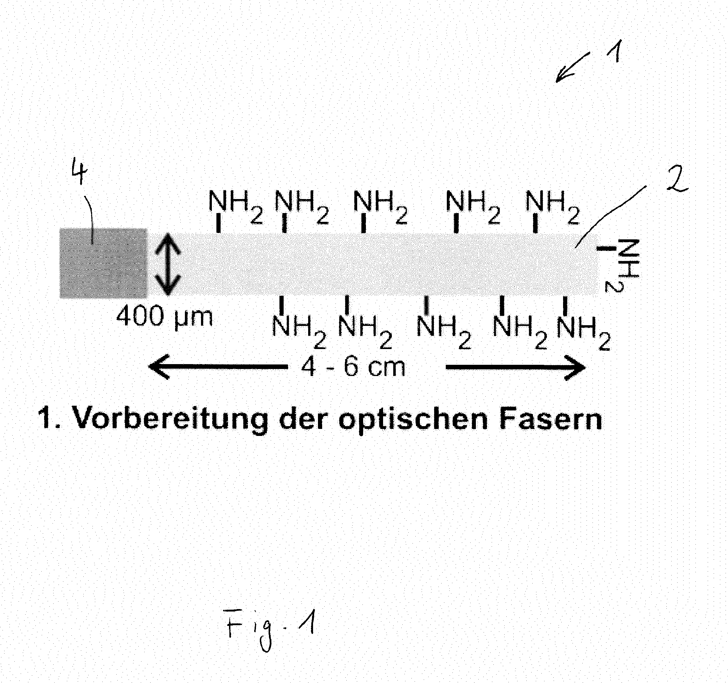

In Figur 1 ist eine schematische Darstellung des Detektionskatheters 1 vor Aufbringung einer Funktionsschicht gezeigt. Gemäß der Figur 1 weist die Detektionsvorrichtung 1 eine als Träger 2 ausgebildete Messstruktur auf. Die Messstruktur 2 kann durch eine oder mehrere optische Fasern ausgebildet sein. Der funktionalisierbare Abschnitt der Messstruktur 2 kann beispielsweise eine Länge von 4 bis 6 cm aufweisen. Die Gesamtheit der optischen Fasern kann einen Durchmesser von etwa 400 µm aufweisen. Ebenso kann eine einzelne Faser mit einem Durchmesser von etwa 400 µm vorgesehen sein. Am proximalen Ende der Detektionsvorrichtung 1 kann die Messstruktur 2 von einer Halterung 4 umgeben sein.

Auf der Oberfläche der Messstruktur 2 kann ein Haftvermittler vorgesehen sein, der beispielsweise Aminogruppen und/oder Carboxylgruppen aufweisen kann. In die Figur 1 ist der Haftvermittler schematisch mit der Angabe NH₂ (Aminogruppen) angedeutet. Es ist auch möglich ohne Haftvermittler die Anbindung einer Funktionsschicht auf der Messstruktur 2 zu erzielen.

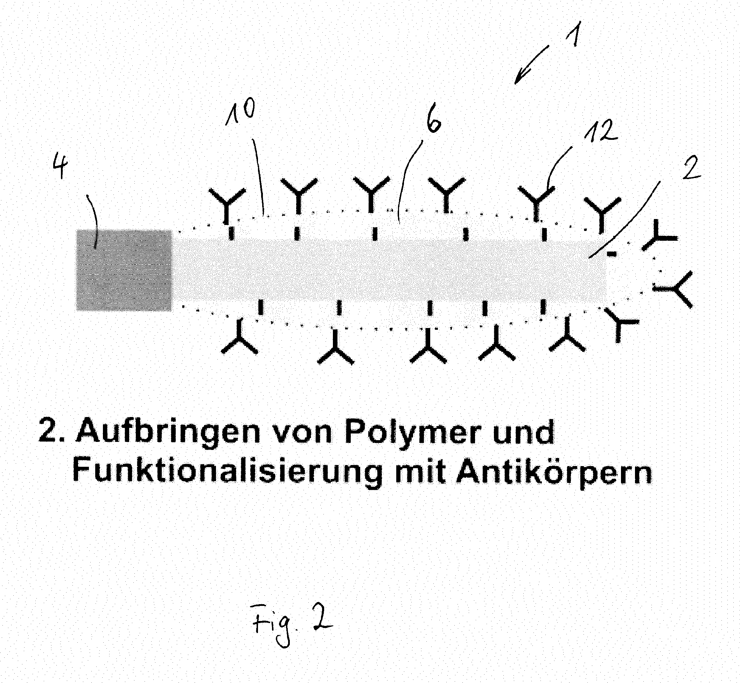

Figur 2 zeigt ein Ausführungsbeispiel der Detektionsvorrichtung 1 nach Aufbringung einer Funktionsschicht 6. Die Funktionsschicht 6 kann beispielsweise aus einem Polymer bestehen. Auf der Funktionsschicht 6 ist eine Funktionsfläche 10 ausgebildet, die mit Detektionsrezeptoren 12 bestückt ist. Die Funktionsfläche 10 kann jedoch auch unmittelbar auf der Messstruktur 2 ausgebildet sein.

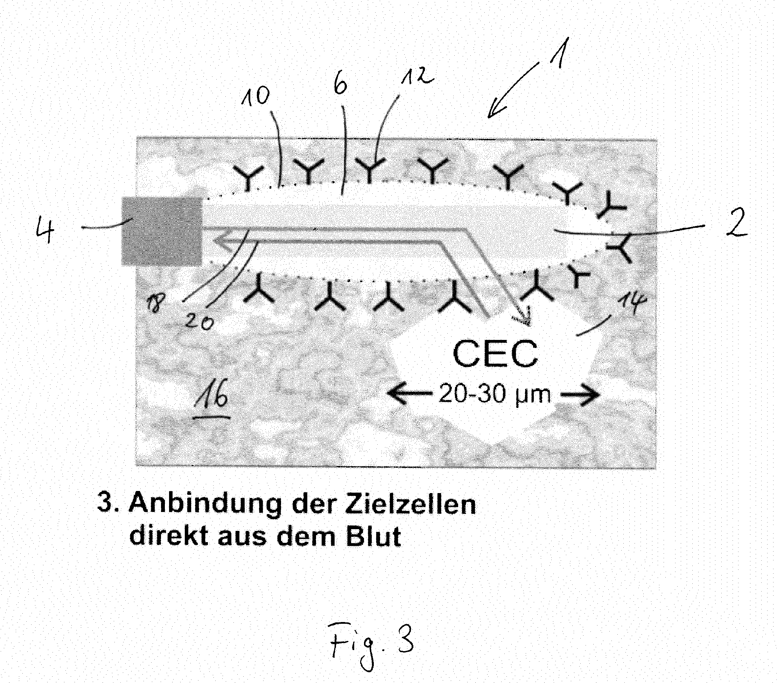

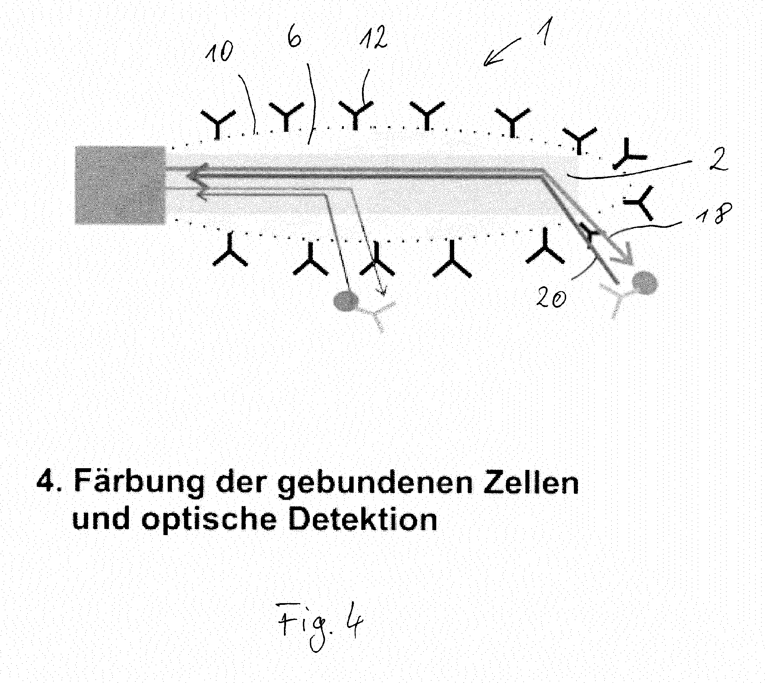

In Figur 3 ist schematisch dargestellt, dass durch Detektionsrezeptoren 12 Zielmoleküle 14 aus Blut 16 gebunden werden. Durch die Pfeile 18 und 20 ist angedeutet, dass die gebundenen Zielmoleküle 14 mittels der Messstruktur 2 optisch gemessen werden können. Insbesondere besteht die Möglichkeit, auf diese Weise die Anzahl der gebundenen Zielmoleküle beziehungsweise Zielzellen zu ermitteln. Wie in der Figur 4 dargestellt, kann es für die optische Messung, insbesondere für die Fluoreszensmessung erforderlich sein, die gebundenen Zielmoleküle beziehungsweise Zielzellen einzufärben und anschließend die optische Detektion vorzunehmen.

Wie voranstehend beschrieben kann die Funktionsschicht 6 aus einem Polymer, insbesondere einem biokompatiblen Polymer bestehen. Das biokompatiblen Polymer ist vorzugsweise ein Hydrogel mit kohlenstoffhaltigen langen verzweigten Makromolekülen, welche über eine hohe Anzahl an funktionellen Gruppen, z.B. Carboxyl-Gruppen bzw. Polycarboxylaten, verfügen. Die Art der funktionellen Gruppen richtet sich nach den Moleküleigenschaften der spezifischen Detektionsrezeptoren 12. Das biokompatible Hydrogel gewährleistet dadurch die dauerhafte kovalente Bindung der Detektionsrezeptoren 12 unter Erhalt der biologischen Funktion und verhindert gleichzeitig, dass die Detektionsrezeptoren 12 durch unspezifische Adsorptionsphänomene in ihrer Erkennungsfunktion beeinträchtigt werden. Hydrogele sind dreidimensional vernetzte hydrophile Polymere, die Flüssigkeiten wie z.B. Wasser aufnehmen, selbst aber darin unlöslich sind. Hauptbestandteile des Hydrogels sind Polyacrylsäure (PAA) und Polyethylenglycol (PEG).

Über eine geeignete Auswahl der Monomerbausteine, des Vernetzungsgrades und der Vernetzungsdichte lassen sich Eigenschaftsprofile je nach gewünschten Anforderungen oder Einsatzbereiche maßschneidern. Eine wesentliche Eigenschaft ist die Biokompatibilität, d. h. die Verträglichkeit des Hydrogels mit dem lebenden Gewebe. Durch die verzweigten Polymerketten des biokompatiblen Polymers wird aber auch die thrombogene Wirkung während der invasiven Anwendung unterbunden. Durch chemische Aktivierung erhalten die funktionellen Gruppen eine unausgeglichene Molekülladung, die es ermöglicht, gelöste Detektionsrezeptoren 12 aus einer Lösung elektrostatisch anzuziehen und kovalent zu binden. Die dauerhaft an der Polymerschicht immobilisierten Detektionsrezeptoren 12 dienen der spezifischen Bindung der Liganden beziehungsweise Zielmoleküle und Zielzellen über ihre Oberflächenantigene und ermöglichen so die Funktion der Detektionsvorrichtung 1. Zusätzlich können in diesem biokompatiblen Polymer chemisch oder enzymatisch spaltbare Gruppen enthalten sein, um die quantitative Gewinnung von gebundenen Zielmolekülen oder Zellen zu vereinfachen.

Die verzweigten Molekülstrukturen des biokompatiblen Polymers 6 können eine im mikroskopischen und/oder im sichtbaren Bereich dreidimensional strukturierte Funktionsfläche 10 mit einander zugewandten Funktionsabschnitten und von Probenflüssigkeit durchsetzbaren Zwischenräumen bilden.

Zum Konservieren und zum Schutz vor den Bedingungen der Endsterilisation sowie zum Strahlenschutz und zur Haltbarkeit des Produkts kann über das biokompatible Polymer 6 eine hier nicht gezeigte biokompatible Schutzschicht (tertiäre Schicht bzw. Stabilisatorschicht) aufgebracht werden. Diese Schutzschicht trocknet über der Funktionsschicht ein und bildet ein dichtes Netz aus kristallinen Strukturen und stabilisiert und konserviert somit den funktionellen Teil der Detektionsvorrichtung 1. Die Schutzschicht ist nicht kovalent gebunden. Im Blutstrom geht die Schutzschicht in Lösung und gibt die Funktionsfläche 10 des Katheters frei. Alternativ kann die Schutzschicht vor der Anwendung mit sterilem Wasser abgewaschen werden.

Die Schutzschicht kann hochreine Alginate, Polyethylenglykole, zyklische und nicht zyklische Oligosaccharide und Polysaccharide, antioxidative Aminosäuren, Proteine und Vitamine umfassen. Die Schutzschicht besteht vorzugsweise aus einem biokompatiblen hochviskosen Polysaccharid, welches als Medium für zugesetzte Aminosäuren, Proteinen, Vitaminen und stabilisierende Polysaccharide dient. Die hohe Viskosität erlaubt eine rasche Benetzbarkeit der Oberfläche. Die angelagerte Schutzschicht klebt an der Sekundärbeschichtung und verhindert das Eindringen von Fremdsubstanzen während einer Lagerung. Die zugesetzten Aminosäuren, Proteine und Vitamine sind im Vergleich zu den spezifischen Liganden in höheren Konzentrationen vorhanden und dadurch in der Lage, die Wahrscheinlichkeit einer Schädigung der Zielmoleküle durch radikale Moleküle oder Ladungsträger auf sich zu lenken bzw. abzufangen und durch Rekombinationsprozesse zerstörte chemische Bindungen wiederherzustellen.

Der fertig gestellte Katheter 1 wird in keimarmer Umgebung verpackt. Die Endsterilisation erfolgt mittels Gamma-Bestrahlung bei einer Strahlungsdosis von 25 kGy. Der Katheter 1 ist für die einmalige Anwendung vorgesehen.

### Anwendung der Detektionsvorrichtung

Der erfindungsgemäß hergestellte Katheter 1 mit Messstruktur 2, Funktionsfläche 10 und gekoppelten Detektionsrezeptoren 12 eignet sich für die Gewinnung seltener Zellen aus dem Blutkreislauf. Hierzu zählen folgende Anwendungsbeispiele:
- Gewinnung von Zellen, umfassend bevorzugt zirkulierende Endothelzellen oder disseminierte Tumorzellen, insbesondere von hämatogen metastasierenden Tumoren z.B. mit dem humanisierten Antikörper Anti-EpCAM, welcher das für viele Krebszellen typische Zelloberflächenprotein EpCAM erkennt.
- Gewinnung und/oder Elmination von Tumor- oder Biomarkern, Toxinen, Arzneimitteln, radioaktiven Tracern und/oder Magnetobeads.
- Gewinnung von embryonalen Trophoblasten aus dem mütterlichen Blutkreislauf mit z.B.
   spezifischen Antikörperfragmenten (F(ab)-Fragmenten) und murinen monoklonalen Antikörpern (lgG), welche das für Trophoblasten typische Zelloberflächenprotein HLA-G erkennen können.

Eine mögliche Anwendung der Detektionsvorrichtung 1 liegt in der pränatalen und Krebs-Diagnostik. Die Detektionsvorrichtung 1 kann beispielsweise dafür eingesetzt werden, im Blutkreislauf von Schwangeren oder Krebspatienten zirkulierende fetale Zellen oder Tumorzellen zu isolieren. Zur Anwendung wird die Detektionsvorrichtung 1 über ein geeignetes kommerziell erhältliches Braunülensystem in die Vene eingeführt und in den venösen Blutkreislauf appliziert. Die Verweildauer in der Vene kann ca. 30 min betragen. Nach der Entnahme der Detektionsvorrichtung 1 aus der Blutbahn werden die auf der Detektionsvorrichtung 1 gebundenen Zellen mittels gezielter Labordiagnostik weiter angereichert und molekularbiologisch sowie zellbiologisch charakterisiert.

Ziel des durchzuführenden minimal invasiven Verfahrens ist die Selektion von zirkulierende Endothelzellen, Tumorzellen oder fetalen aus dem Blut. Auf Grund der niedrigen Zellkonzentration der Zellen im Blut wäre eine Blutentnahme von ca. 0,5 l notwendig, um die gewünschte Zielzellzahl zu erhalten. Dies ist aus medizinischer Sicht jedoch ausgeschlossen.

Zirkulierende Endothelzellen können mit einem gegen CD-164 gerichteten Antikörper angereichert werden, der kovalent an das Hydrogel gebunden ist. Krebstumorzellen können mit dem EpCAM Antikörper (gegen das EpCAM Antigen) angereichert werden, der in seinen konstanten Domänen humanisiert ist und kovalent an das Hydrogel gebunden ist. Fetale Trophoblasten können mit einem gegen HLA-G Antikörper aus dem Mutterblut angereichert werden, der kovalent an das Hydrogel gebunden ist.

Aufgrund der Anordnung der Messstruktur kann auf die voranstehend beschrieben Verwendung der Detektionsvorrichtung vereinfacht werden, da mit nur geringem Zeitaufwand bestimmt werden kann, ob die erforderliche Anzahl an Liganden beziehungsweise Zielmolekülen oder Zielzellen gebunden wurde oder ob weitere Gewinnung von Probenmaterial erforderlich ist.

Die beschriebene Detektionsvorrichtung 1 eignet sich zur Anreicherung beziehungsweise Aufkonzentrierung von Zellen oder Biomolekülen aus Körperflüssigkeiten oder anderen flüssigen Analyseproben. Die flüssigen Untersuchungsproben werden in die Detektionsvorrichtung 1 appliziert oder werden sofort mittels Luer-Lock Entnahme- beziehungsweise Probenahmesysteme eingeleitet. Das isolierte und angereicherte Material kann sofort einer nachgeschalteten Diagnostik, beispielweise über eine Lab-on-Chip Technologie, zugeführt werden.

Diese Detektionsvorrichtung 1 zeichnet sich aufgrund der Konstruktion durch sehr einfache Handhabung aus, welche ohne großen Zeitaufwand mittels eines Kits in jeder diagnostischen oder klinischen Einrichtung verwendet werden kann.

## Patentansprüche

1. Detektionsvorrichtung (1) zur Anreicherung von Probenmaterial, umfassend eine mit Detektionsrezeptoren (12) bestückte Funktionsfläche (10), wobei die Funktionsfläche (10) mittelbar oder unmittelbar auf einer Messstruktur angeordnet ist, die ein optisch leitendes Material zur optischen Messung von an den Detektionsrezeptoren (12) angereicherten Zielmolekülen aufweist, wobei die mit den Detektionsrezeptoren (12) bestückte Funktionsfläche (10) durch eine Funktionsschicht aus hämokompatiblem Material gebildet ist, die entlang des Funktionsabschnitts der Detektionsvorrichtung mittelbar oder unmittelbar auf der Messstruktur angeordnet ist, wobei das hämokompatible Material als Hydrogel ausgebildet ist, **dadurch gekennzeichnet, dass** die Funktionsfläche (10) mit einer in Flüssigkeiten löslichen Schutzschicht (4) überzogen ist.

2. Detektionsvorrichtung (1) nach Anspruch 1, **dadurch gekennzeichnet, dass** die Messstruktur einen Lichtwellenleiter aus einem Kunststoffmaterial aufweist und/oder dass die Messstruktur eine optische Faser, vorzugsweise eine polymere optische Faser aufweist, wobei die optische Faser vorzugsweise aus einem nicht-fluoreszierendem Material besteht, insbesondere CYTOP, Fontex®, PMMA.

3. Detektionsvorrichtung (1) nach wenigstens einem der vorangegangenen Ansprüche, **dadurch gekennzeichnet, dass** die Messstruktur eine einzelne oder mehrere miteinander verbundene und/oder verdrehte Fasern aufweist und/oder dass die Messstruktur einen durchgängigen Kern bildet und/oder dass die Messstruktur als Träger für die Funktionsfläche (10) ausgebildet ist.

4. Detektionsvorrichtung (1) nach wenigstens einem der vorangegangenen Ansprüche, **dadurch gekennzeichnet, dass** die Messstruktur an ihrem proximalen Ende einen Anschluss für ein optisches Messgerät, vorzugsweise für ein fasergekoppeltes Spektrometer oder eine Photodiodeneinheit aufweist.

5. Detektionsvorrichtung (1) nach wenigstens einem der vorangegangenen Ansprüche, **dadurch gekennzeichnet, dass** die Messstruktur an ihrem proximalen Ende abschnittsweise von einer Halterung umgeben ist.

6. Detektionsvorrichtung (1) nach wenigstens einem der vorangegangenen Ansprüche, **dadurch gekennzeichnet, dass** die Detektionsrezeptoren (12) Antikörper, Antikörperfragmente, Aminosäuren-Strukturen, Nukleinsäure-Strukturen und/oder synthetische Strukturen mit spezifischer Affinität zu Oberflächen von Zielzellen umfassen, vorzugsweise monoklonale Antikörper murinen Ursprungs, chimäre Antikörper oder humanisierte Antikörper, bevorzugt anti-CD146 und/oder anti-EpCAM Antikörper.

7. Detektionsvorrichtung (1) nach wenigstens einem der vorangegangenen Ansprüche, **dadurch gekennzeichnet, dass** sich die Funktionsfläche (10) über einen Funktionsabschnitt erstreckt, der am distalen Ende der Detektionsvorrichtung (1) ausgebildet ist, wobei der Funktionsabschnitt vorzugsweise eine Länge von 20-60 mm und/oder eine Dicke von 0,1 bis 1,0 mm, besonders bevorzugt von 0,5 mm aufweist.

8. Detektionsvorrichtung (1) nach wenigstens einem der vorangegangenen Ansprüche, **dadurch gekennzeichnet, dass** die Funktionsfläche (10) abschnittsweise oder durchgängig mit Detektionsrezeptoren (12) bestückt ist und/oder dass die Funktionsfläche (10) mit Detektionsrezeptoren (12) unterschiedlicher Art oder unterschiedlicher Spezifitäten bestückt ist.

9. Detektionsvorrichtung (1) nach wenigstens einem der vorangegangenen Ansprüche, **dadurch gekennzeichnet, dass** an den Detektionsrezeptoren (12) angereicherte Zielmoleküle mittels eines fluoreszenzmarkierten Antikörpers markierbar sind.

10. Detektionsvorrichtung (1) nach wenigstens einem der vorangegangenen Ansprüche, **dadurch gekennzeichnet, dass** an den Detektionsrezeptoren (12) angereicherte Zielmoleküle mittels der Messstruktur fluoreszenzspektroskopisch durch Bestimmung einer Wellenlängenverschiebung und/oder Intensitätsänderung und/oder einer Änderung der Lumineszenzabklingzeit detektierbar sind.

11. Detektionsvorrichtung (1) nach wenigstens einem der vorangegangenen Ansprüche, **dadurch gekennzeichnet, dass** die Schutzschicht in Blut löslich ist und/oder biokompatibel ist.

12. Detektionsvorrichtung (1) nach wenigstens einem der vorangegangenen Ansprüche, **dadurch gekennzeichnet, dass** die Detektionsrezeptoren (12) über Linker oder organische funktionelle Gruppen mit der Funktionsfläche (10) wirkverbunden sind, bevorzugt durch chemische Bindung, besonders bevorzugt durch kovalente Bindung und/oder dass die Detektionsrezeptoren (12) über Carboxylgruppen an der Funktionsfläche (10) gekoppelt sind, wobei die Kopplung der Detektionsrezeptoren (12) über Carbodiimidchemie und/oder UV-Quervernetzung erfolgt und/oder wobei die Carboxylgruppen an der Messstruktur oder innerhalb der Funktionsschicht vorgesehen sind.

13. Detektionsvorrichtung (1) nach wenigstens einem der vorangegangenen Ansprüche, hergestellt durch:
a. Bereitstellung einer Messstruktur mit einem Funktionsabschnitt, und
b. Funktionalisierung des Funktionsabschnitts mit Detektionsrezeptoren zur Ausbildung einer Funktionsschicht, wobei die Kopplung der Detektionsrezeptoren vorzugsweise über Carbodiimidchemie erfolgt.

14. Verwendung einer Detektionsvorrichtung (1) nach wenigstens einem der vorangegangenen Ansprüche zur in vitro Anreicherung von Probenmaterial, insbesondere zur Anreicherung von Zellen, umfassend bevorzugt zirkulierende Endothelzellen oder disseminierte Tumorzellen, insbesondere von hämatogen metastasierenden Tumoren, oder zur Elimination von Arzneimittel, zur Elimination von radioaktiven Tracern, zur Elimination von Magnetobeads, zur Gewinnung von Tumor- oder Biomarkern und/oder zur Elimination von Toxinen.

15. Verfahren zur Anreicherung von Probenmaterial in vitro unter Benutzung einer Detektionsvorrichtung (1) nach wenigstens einem der Ansprüche 1 bis 13, wobei das Verfahren die folgenden Schritte umfasst:
a. Beaufschlagen der Funktionsfläche (10) mit einer Probenflüssigkeit,
b. Anreicherung von Probenmaterial, vorzugsweise Zellen, DNA, RNA, Proteine, Peptide, synthetische Moleküle, an den Detektionsrezeptoren (12)
c. Markierung des an den Detektionsrezeptoren (12) angereicherten Probenmaterials, vorzugsweise mit einem fluoreszenzmarkierten Antikörper
d. Optische Detektion des Probenmaterials durch die Messstruktur mittels faseroptischer Messung, vorzugsweise Fluoreszensmessung.

## Claims

1. A detection device (1) for the enrichment of sample material, comprising a functional surface (10) equipped with detection receptors (12), the functional surface (10) being arranged directly or indirectly on a measuring structure which contains an optically conductive material for the optical measurement of target molecules enriched on the detection receptors (12), wherein the functional surface (10) equipped with the detection receptors (12) is formed by a functional layer made of hemocompatible material which is arranged directly or indirectly along the functional section of the detection device on the measuring structure, wherein the hemocompatible material is formed as a hydrogel, **characterized in that** the functional surface (10) is coated with a protective layer (4) soluble in liquids.

2. The detection device (1) according to claim 1, **characterized in that** the measuring structure has an optical waveguide made of a plastic material and / or that the measuring structure has an optical fiber, preferably a polymeric optical fiber, the optical fiber preferably consisting of a non-fluorescent material, in particular CYTOP, Fontex®, PMMA.

3. The detection device (1) according to at least one of the preceding claims, **characterized in that** the measuring structure has a single or several interconnected and / or twisted fibers and / or that the measuring structure forms a continuous core and / or that the measuring structure is formed as a carrier for the functional surface (10).

4. The detection device (1) according to at least one of the preceding claims, **characterized in that** the measuring structure has a connection for an optical measuring device at its proximal end, preferably for a fiber-coupled spectrometer or a photodiode unit.

5. The detection device (1) according to at least one of the preceding claims, **characterized in that** the measuring structure is surrounded in sections by a holder at its proximal end.

6. The detection device (1) according to at least one of the preceding claims, **characterized in that** the detection receptors (12) comprise antibodies, antibody fragments, amino acid structures, nucleic acid structures and / or synthetic structures with specific affinity for surfaces of target cells, preferably monoclonal antibodies of murine origin, chimeric antibodies or humanized antibodies, preferably anti-CD146 and / or anti-EpCAM antibodies.

7. The detection device (1) according to at least one of the preceding claims, **characterized in that** the functional surface (10) extends over a functional section formed at the distal end of the detection device (1), the functional section preferably having a length of 20-60 mm and / or a thickness of 0.1 to 1.0 mm, particularly preferably 0.5 mm.

8. The detection device (1) according to at least one of the preceding claims, **characterized in that** the functional surface (10) is equipped with detection receptors in sections or continuously (12) and / or that the functional surface (10) is equipped with detection receptors (12) of different types or different specificities.

9. The detection device (1) according to at least one of the preceding claims, **characterized in that** target molecules enriched at the detection receptors (12) can be labeled by means of a fluorescently labeled antibody.

10. The detection device (1) according to at least one of the preceding claims, **characterized in that** target molecules enriched at the detection receptors (12) can be detected by fluorescence spectroscopy by means of the measuring structure by determining a wavelength shift and / or a change in intensity and / or a change in the luminescence decay time.

11. The detection device (1) according to at least one of the preceding claims, **characterized in that** the protective layer is soluble in blood and / or is biocompatible.

12. The detection device (1) according to at least one of the preceding claims, **characterized in that** the detection receptors (12) are operatively connected with the functional surface (10) via linkers or organic functional groups, preferably by chemical bonding, particularly preferably by covalent bonding and / or that coupling of the detection receptors (12) to the functional surface (10) is carried out via carboxyl groups, wherein the detection receptors (12) are coupled via carbodiimide chemistry and / or UV cross-linking and / or wherein the carboxyl groups are provided on the measuring structure or within the functional layer.

13. The detection device (1) according to at least one of the preceding claims, manufactured by:
a. providing a measuring structure with a functional section, and
b. functionalizing the functional section with detection receptors to form a functional layer, wherein coupling of the detection receptors is preferably carried out via carbodiimide chemistry.

14. Use of a detection device (1) according to at least one of the preceding claims for the in vitro enrichment of sample material, in particular for the enrichment of cells, preferably comprising circulating endothelial cells or disseminated tumor cells, in particular of hematogenous metastastatic tumors, or for the elimination of drugs, for the elimination of radioactive tracers, for the elimination of magnetobeads, for obtaining tumor markers or biomarkers and / or for the elimination of toxins.

15. Method for the enrichment of sample material in vitro using a detection device (1) according to at least one of claims 1 to 13, the method comprising the following steps:
a. loading a sample liquid onto the functional surface (10),
b. enrichment of sample material, preferably cells, DNA, RNA, proteins, peptides, synthetic molecules, at the detection receptors (12)
c. labeling the sample material enriched at the detection receptors (12), preferably with a fluorescently labeled antibody
d. optical detection of the sample material by the measuring structure by means of fiber optic measurement, preferably fluorescence measurement.

## Revendications

1. Dispositif de détection (1) pour l'enrichissement d'une matière d'échantillon, comportant une surface fonctionnelle (10) équipée de récepteurs de détection (12), dans lequel la surface fonctionnelle (10) est agencée directement ou indirectement sur une structure de mesure, qui comporte un matériau optiquement conducteur pour la mesure optique de molécules cible enrichies sur les récepteurs de détection (12), dans lequel la surface fonctionnelle (10) équipée de récepteurs de détection (12) est constituée d'une couche fonctionnelle en matériau hémocompatible, qui est agencé directement ou indirectement sur la structure de mesure le long de la section fonctionnelle du dispositif de détection, dans lequel le matériau hémocompatible est constitué comme un hydrogel, **caractérisé en ce que** la surface fonctionnelle (10) est recouverte d'une couche de protection (4) soluble dans les fluides.

2. Dispositif de détection (1) selon la revendication 1, **caractérisé** en que la structure de mesure comporte un guide d'onde lumineuse en matériau synthétique et/ou en ce que la structure de mesure comporte une fibre optique, de préférence une fibre optique polymère, dans lequel la fibre optique est constituée de préférence d'un matériau non fluorescent, en particulier le CYTOP, le Fontex® ou le PMMA.

3. Dispositif de détection (1) selon au moins l'une des revendications précédentes, **caractérisé en ce que** la structure de mesure comporte une ou plusieurs fibres torsadées et/ou liées entre elles, et/ou **en ce que** la structure de mesure constitue une âme continue, et/ou **en ce que** la structure de mesure est constituée comme un support pour la surface fonctionnelle (10).

4. Dispositif de détection (1) selon au moins l'une des revendications précédentes, **caractérisé en ce que** la structure de mesure comporte à son extrémité proximale une connexion pour un appareil de mesure optique, de préférence pour un spectromètre couplé par fibre ou une unité à photodiode.

5. Dispositif de détection (1) selon au moins l'une des revendications précédentes, **caractérisé en ce que** la structure de mesure est entourée d'un support sur une section à son extrémité proximale.

6. Dispositif de détection (1) selon au moins l'une des revendications précédentes, **caractérisé en ce que** les récepteurs de détection (12) comportent des anticorps, des fragments d'anticorps, des structures à acides aminés, des structures à acides nucléiques et/ou des structures synthétiques avec une affinité spécifique pour les surfaces de cellules cible, de préférence des anticorps monoclonaux d'origine murine, des anticorps chimériques ou des anticorps humanisés, de préférence des anticorps anti-CD146 et/ou anti-EpCAM.

7. Dispositif de détection (1) selon au moins l'une des revendications précédentes, **caractérisé en ce que** la surface fonctionnelle (10) s'étend sur une section fonctionnelle agencée à l'extrémité distale du dispositif de détection (1), dans lequel la section fonctionnelle présente de préférence une longueur de 20 à 60 mm et/ou une épaisseur de 0,1 à 1,0 mm, et particulièrement de préférence de 0,5 mm.

8. Dispositif de détection (1) selon au moins l'une des revendications précédentes, **caractérisé en ce que** la surface fonctionnelle (10) est équipée sur certaines sections ou en continu de récepteurs de détection (12), et/ou **en ce que** la surface fonctionnelle (10) est équipée de récepteurs de détection (12) de différents types ou de différentes caractéristiques.

9. Dispositif de détection (1) selon au moins l'une des revendications précédentes, **caractérisé en ce que** les récepteurs de détection (12) peuvent être marqués par des molécules cible enrichies au moyen d'un anticorps marqué par fluorescence.

10. Dispositif de détection (1) selon au moins l'une des revendications précédentes, **caractérisé en ce que** des molécules cible enrichies peuvent être détectées sur les récepteurs de détection (12) au moyen de la structure de mesure par spectroscopie de fluorescence moyennant la détermination d'un décalage de longueur d'onde et/ou d'un changement d'intensité et/ou d'un changement de temps de décroissance de luminescence.

11. Dispositif de détection (1) selon au moins l'une des revendications précédentes, **caractérisé en ce que** la couche de protection est soluble dans le sang et/ou est biocompatible.

12. Dispositif de détection (1) selon au moins l'une des revendications précédentes, **caractérisé en ce que** les récepteurs de détection (12) sont liés fonctionnellement à la surface fonctionnelle (10) via des liaisons ou des groupes fonctionnels organiques, de préférence via une liaison chimique, et particulièrement de préférence via une liaison covalente, et/ou **en ce que** les récepteurs de détection (12) sont couplés à la surface fonctionnelle (10) via des groupes carboxyle, dans lequel le couplage des récepteurs de détection (12) se produit par chimie carbodiimide et/ou par réticulation sous UV, et/ou dans lequel les groupes carboxyle sont pourvus sur la structure de mesure ou dans la couche fonctionnelle.

13. Dispositif de détection (1) selon au moins l'une des revendications précédentes, constitué par :
a. la préparation d'une structure de mesure avec une section fonctionnelle, et
b. la fonctionnalisation de la section fonctionnelle avec des récepteurs de détection pour la constitution d'une couche fonctionnelle, dans lequel le couplage des récepteurs de détection se produit de préférence par chimie carbodiimide.

14. Utilisation d'un dispositif de détection (1) selon au moins l'une des revendications précédentes pour l'enrichissement in vitro d'une matière d'échantillon, en particulier pour l'enrichissement de cellules, comprenant de préférence des cellules endothéliales circulantes ou des cellules tumorales disséminées, en particulier des tumeurs métastasiantes transmises par le sang, ou pour l'élimination de médicaments, pour l'élimination de traceurs radioactifs, pour l'élimination de billes magnétiques, pour la récupération de marqueurs tumoraux ou de biomarqueurs, et/ou pour l'élimination de toxines.

15. Procédé d'enrichissement in vitro d'une matière d'échantillon en utilisant un dispositif de détection (1) selon au moins l'une des revendications 1 à 13, dans lequel le procédé comprend les étapes suivantes :
a. application d'un fluide d'échantillon sur la surface fonctionnelle (10),
b. enrichissement de la matière d'échantillon, de préférence constituée de cellules, d'ADN, d'ARN, de protéines, de peptides ou de molécules synthétiques, sur les récepteurs de détection (12),
c. marquage de la matière d'échantillon enrichie sur les récepteurs de détection (12), de préférence avec un anticorps marqué par fluorescence,
d. détection optique de la matière d'échantillon par la structure de mesure au moyen d'une mesure par fibre optique, et de préférence d'une mesure de fluorescence.
